# EUROPEAN PATENT APPLICATION

(11) **EP 3 015 466 A1**
(43) Date of publication of application: **04.05.2016**
(21) Application number: 14817850.2
(22) Date of filing: 26.05.2014
(51) Int. Cl.: C07D 405/06

(54) **METHOD FOR PRODUCING AZOLE DERIVATIVE**

(30) Priority: 26.06.2013 JP 2013134303
(71) Applicant: Kureha Corporation, Chuo-ku Tokyo 103-8552 (JP)
(72) Inventor: MASANO, Taiga, Tokyo 103-8552 (JP); SUDO, Keiichi, Tokyo 103-8552 (JP)
(74) Representative: Hoefer & Partner Patentanwälte mbB
(86) International application number: PCT/JP2014/063861
(87) International publication number: WO 2014/208243

(57) **Abstract**

In the present invention, a compound (I) is reacted with a base selected from the group consisting of a metal alkoxide, a metal hydride compound, an alkali metal, an organometallic compound of an alkali metal, an alkali metal amide, an alkali metal carbonate salt, an alkali earth metal carbonate salt, and a mixture of these to produce a compound (II). Thereafter, this reaction solution is mixed with a compound (III) and a sulfonium compound or a sulfoxonium compound in the presence of a base to obtain an azole derivative (V).

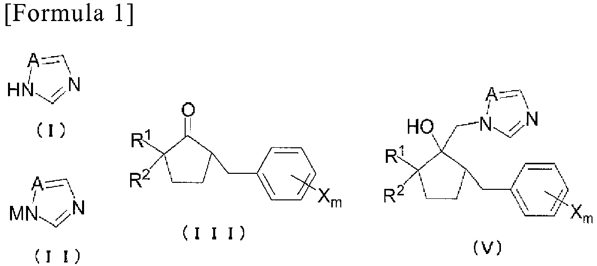

## Description

### TECHNICAL FIELD

The present invention relates to a method for producing an azole derivative.

### BACKGROUND ART

Some known azolylmethylcyclopentanol derivatives exhibit excellent activity as active ingredients in agricultural and horticultural disease control agents and agents for protecting industrial materials. For example, Patent Document 1 discloses a 2-(halogenated hydrocarbon-substituted)-5-benzyl-1-azolylmethylcyclopentanol derivative, which exhibits low toxicity to humans and animals and which exhibits a high controlling effect on a wide range of plant diseases and a high growth promoting effect on a variety of agricultural and horticultural plants.

Patent Document 1 also discloses azolylmethylation of a 2-substituted-5-benzylcyclopentanone derivative, which is an intermediate product, as a part of a production step of 2-(halogenated hydrocarbon substituted)-5-benzyl-1-azolylmethylcyclopentanol derivative.

### CITATION LIST

### Patent Literature(s)

Patent Document 1: WO/2011/070771 (published on June 16, 2011)

### SUMMARY OF INVENTION

### Technical Problem

A triazole sodium salt used in the azolylmethylation described above is produced by dissolving triazole and sodium hydroxide in a mixed solvent of an aprotic polar solvent, such as N,N-dimethylacetamide, toluene, and water to perform azeotropic dehydration of water using toluene. The generated triazole sodium salt is used for the azolylmethylation as a suspension for the polar solvent described above or as a solid after filtration, after distilling off the toluene under reduced pressure.

However, since toluene and water are not the necessary solvents for the azolylmethylation reaction described above, it is preferable to reduce toluene and water. Furthermore, the azeotropic dehydration step requires some time. When water remains in the suspension or solid of the triazole sodium salt, efficiency of the azolylmethylation reaction may decrease.

In the light of the above problems, an object of the present invention is to provide a method that does not use water and that can achieve simplification of the procedure in azolylmethylation of a 2-substituted-5-benzylcyclopentanone derivative.

### Solution to Problem

The present invention is a method for producing an azole derivative represented by general formula (V) below: 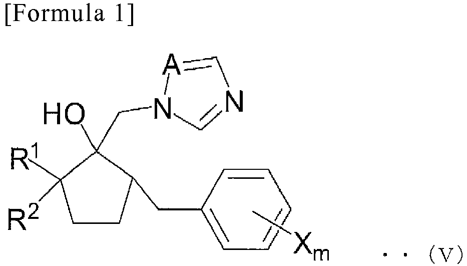 (in formula (V), R¹ and R² each independently represent a hydrogen atom, an alkyl group having from 1 to 4 carbons, or -CₙH₂ₙ-O-G; G represents a protecting group; n represents an integer of 1 to 4; R¹ and R² may together form a ring; X represents a halogen atom, an alkyl group having from 1 to 4 carbons, a haloalkyl group having from 1 to 4 carbons, an alkoxy group having from 1 to 4 carbons, a haloalkoxy group having from 1 to 4 carbons, a phenyl group, a cyano group, or a nitro group; m represents an integer of 0 to 5; when m is 2 or greater, a plurality of X may be different from each other; and A represents a nitrogen atom or a methine group);
the method comprising:
a step 1 of obtaining a reaction solution containing a compound represented by general formula (II) below by reacting a compound represented by general formula (I) below with a base selected from the group consisting of a metal alkoxide, a metal hydride compound, an alkali metal, an organometallic compound of an alkali metal, an alkali metal amide, an alkali metal carbonate salt, an alkali earth metal carbonate salt, and a mixture of these 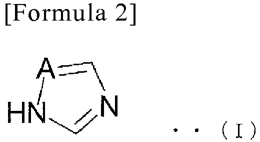 (in formula (I), A is the same as A in formula (V)) 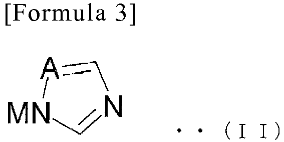 (in formula (II), M represents a metal atom contained in the base used in the step 1; and A is the same as A in formula (V)); and
a step 2 of obtaining an azole derivative represented by general formula (V) above by mixing the reaction solution, a compound represented by general formula (III) below, and a sulfonium compound or a sulfoxonium compound in the presence of a base 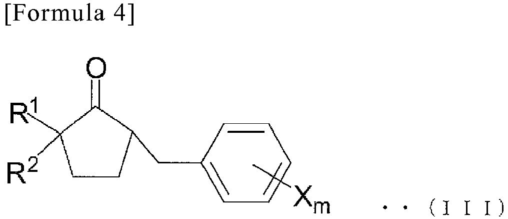 (in formula (III), R1, R2, X, and m are each the same as R1, R2, X, and m in formula (V)).

### Advantageous Effects of Invention

According to the present invention, azolylmethylation of 2-substituted-5-benzylcyclopentanone derivative can be performed without using water while simplification of the procedure is achieved.

### Description of Embodiments

An embodiment of the present invention will be described below.

### 1. Azole Derivative

In the method for producing an azole derivative according to the present embodiment, an azole derivative represented by general formula (V) below (hereinafter, referred to as "azole derivative (V)") is produced. Before explaining the production method in detail, the structure of the azole derivative (V) will be explained below.

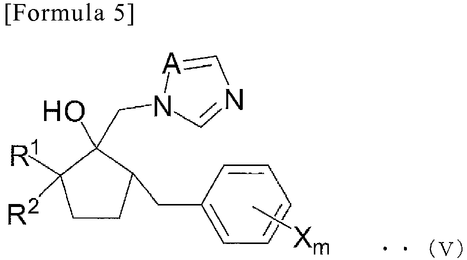

In general formula (V), X is a halogen atom, an alkyl group having from 1 to 4 carbons, a haloalkyl group having from 1 to 4 carbons, an alkoxy group having from 1 to 4 carbons, a haloalkoxy group having from 1 to 4 carbons, a phenyl group, a cyano group, or a nitro group.

Examples of the halogen atom include a chlorine atom, fluorine atom, bromine atom, and iodine atom.

Examples of the alkyl group having from 1 to 4 carbons include a methyl group, ethyl group, n-propyl group, 1-methylethyl group, 2-methylpropyl group, n-butyl group, 1,1-dimethylethyl group, and the like.

Examples of the haloalkyl group having from 1 to 4 carbons include a trifluoromethyl group, 1,1,2,2,2-pentafluoroethyl group, chloromethyl group, trichloromethyl group, bromomethyl group, and the like.

Examples of the alkoxy group having from 1 to 4 carbons include a methoxy group, ethoxy group, n-propoxy group, and the like.

Examples of the haloalkoxy group having from 1 to 4 carbons include a trifluoromethoxy group, difluoromethoxy group, 1,1,2,2,2-pentafluoroethoxy group, 2,2,2-trifluoroethoxy group, and the like.

X is preferably a halogen atom, a haloalkyl group having from 1 to 3 carbons, a haloalkoxy group having from 1 to 3 carbons, an alkyl group having from 1 to 3 carbons, or an alkoxy group having from 1 to 3 carbons, more preferably a halogen atom, a haloalkyl group having from 1 or 2 carbons, or a haloalkoxy group having from 1 or 2 carbons, even more preferably a halogen atom, and particularly preferably a fluorine atom or a chlorine atom.

An integer of 0 to 5 is represented by m. When m is 2 or greater, a plurality of X may be the same or different from each other. Of these, m is preferably an integer of 0 to 3, more preferably an integer of 0 to 2, and even more preferably 0 or 1.

The bonding position of X is not particularly limited; however, in cases where m is 1, a bonding position that forms a 4-substituted benzyl is preferred.

A represents a nitrogen atom or a methine group. A is preferably a nitrogen atom.

R¹ and R² each independently represents a hydrogen atom, alkyl group having from 1 to 4 carbons, or -CₙH₂ₙ-O-G. R¹ and R² may together form a ring.

Examples of the alkyl group having from 1 to 4 carbons include a methyl group, ethyl group, n-propyl group, 1-methylethyl group, 1-methylpropyl group, 2-methylpropyl group, n-butyl group, 1,1-dimethylethyl group, and the like.

In -CₙH₂ₙ-O-G, G represents a protecting group. The protecting group is not particularly limited; however, examples thereof include alkoxymethyl groups, such as a methoxymethyl group and ethoxymethyl group, lower alkyl groups, such as a t-butyl group and methyl group, a substituted or unsubstituted benzyl group, a substituted or unsubstituted tetrahydropyranyl group, a substituted or unsubstituted tetrahydrofuranyl group, an allyl group, and the like. An integer of 1 to 4 is represented by n. Of these, n is preferably an integer of 1 to 3, more preferably 1 or 2, and even more preferably 1. The -CₙH₂ₙ- may be a straight-chain or branched-chain. When both the R¹ and R² are -CₙH₂ₙ-O-G, the two G moieties may together form a ring.

Examples of the protecting group when the two G moieties together form a ring include, but are not limited to, methylene acetal, ethylidene acetal, t-butylmethylidene ketal, 1-t-butylethylidene ketal, 1-phenylethylidene ketal, acrolein acetal, isopropylidene ketal (acetonide), cyclopentylidene ketal, cyclohexylidene ketal, cycloheptylidene ketal, benzylidene acetal, p-methoxybenzylidene acetal, 2,4-dimethoxybenzylidene ketal, 3,4-dimethoxybenzylidene ketal, 2-nitrobenzylidene acetal, 4-nitrobenzylidene acetal, mesitylene acetal, 1-naphthaldehyde acetal, benzophenone ketal, camphor ketal, menthone, methoxymethylene acetal, ethoxymethylene acetal, dimethoxymethylene orthoester, 1-methoxyethylidene orthoester, 1-ethoxyethylidene orthoester, methylidene orthoester, phthalide orthoester, 1,2-dimethoxyethylidene orthoester, α-methoxybenzylidene orthoester, 2-oxacyclopentylidene orthoester, butane-2,3-bis-acetal, cyclohexane-1,2-diacetal, bis-dihydropyran ketal, di-t-butylsilylene, 1,3-(1,1,3,3-tetraisopropyl)disiloxanilidene, 1,1,3,3-tetra-t-butoxydisiloxanilidene, and the like.

R¹ and R² preferably each represent -CₙH₂ₙ-O-G.

Note that, when both the R¹ and R² are -CₙH₂ₙ-O-G, the azole derivative (V) can be represented by general formula (Vc) below.

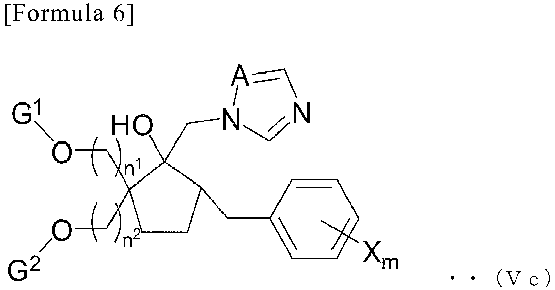

In general formula (Vc), G¹ and G² each represent a protecting group. G¹ and G² may be the same or different from one another. In addition, G¹ and G² may together form a ring. Specific examples of G¹ and G² are the same as G moieties for the case where R¹ and R² of formula (V) are -CₙH₂ₙ-O-G. Furthermore, n¹ and n² each independently represent an integer of 1 to 4 (i.e. 1, 2, 3, or 4). X, m, and A are each the same as X, m, and A in formula (V).

The azole derivative (V) is more preferably an azole derivative represented by general formula (Va) below (hereinafter, referred to as "azole derivative (Va)").

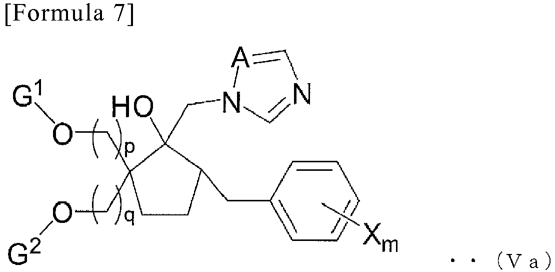

In general formula (Va), p and q are each independently 1 or 2. Preferably, both p and q are 1. X, m, and A are each the same as X, m, and A in formula (V); and G¹ and G² are each the same as G¹ and G² in formula (Vc).

The azole derivative (V) is even more preferably an azole derivative represented by general formula (Vb) below (hereinafter, referred to as "azole derivative (Vb)").

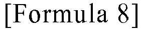

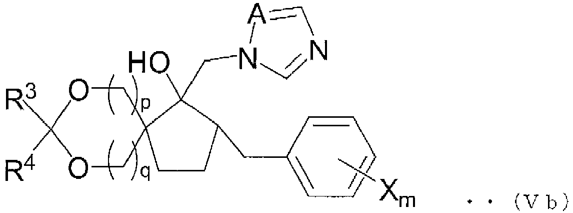

In general formula (Vb), R³ and R⁴ are each independently a hydrogen atom, an alkyl group having from 1 to 4 carbons, a phenyl group, or a benzyl group.

Examples of the alkyl group having from 1 to 4 carbons include a methyl group, ethyl group, n-propyl group, 1-methylethyl group, 1-methylpropyl group, 2-methylpropyl group, n-butyl group, 1,1-dimethylethyl group, and the like.

One or more hydrogen atoms in the phenyl group in R³ and/or R⁴ and one or more hydrogen atoms in the phenyl moiety of the benzyl group in R³ and/or R⁴ may be substituted with alkyl groups having from 1 to 4 carbons, alkoxy groups having from 1 to 4 carbons, or halogen atoms. Examples of the alkyl group having from 1 to 4 carbons as the substituent include a methyl group, ethyl group, n-propyl group, 1-methylethyl group, 1-methylpropyl group, 2-methylpropyl group, n-butyl group, 1,1-dimethylethyl group, and the like. Examples of the alkoxy group having from 1 to 4 carbons as the substituent include a methoxy group, ethoxy group, n-propoxy group, and the like. Examples of the halogen atom as the substituent include a fluorine atom, chlorine atom, bromine atom, and the like.

Of these, R³ and R⁴ are preferably each independently a hydrogen atom or an alkyl group having from 1 to 4 carbons, more preferably an alkyl group having from 1 to 4 carbons, even more preferably an alkyl group having 1 or 2 carbons, and particularly preferably both the R³ and R⁴ are methyl groups.

In general formula (Vb), p, q, A, X, and m are each the same as p, q, A, X, and m in formula (Va).

A preferred example of the azole derivative (V) is an azole derivative represented by general formula (Vd) below; however, the azole derivative (V) is not limited to these.

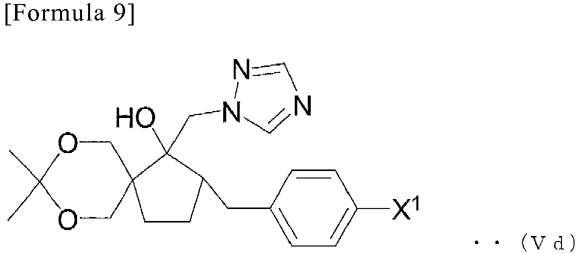

In general formula (Vd), X¹ is a hydrogen atom, a fluorine atom, or a chlorine atom.

The azole derivative (V) can be suitably used to produce an azole derivative represented by general formula (VI) below (hereinafter, referred to as "azole derivative (VI)").

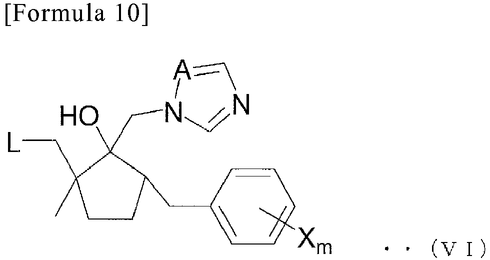

In general formula (VI), X, m, and A are each the same as X, m and A in formula (V). L represents a halogen atom, and a chlorine atom and a bromine atom are preferred as this halogen atom.

The azole derivative (VI) exhibits excellent fungicidal activity against many types of fungi that cause diseases in plants. Moreover, the azole derivative (V) per se also exhibits excellent fungicidal activity against fungi that cause diseases in plants. In addition, the azole derivative (VI) and the azole derivative (V) can also be suitably used as agents for protecting industrial materials and as plant growth regulators.

### 2. Details of method for producing azole derivative (V)

The method for producing the azole derivative (V) according to the present embodiment is a method comprising:
a step of obtaining a reaction solution containing a compound represented by general formula (II) below (hereinafter, referred to as "compound (II)") by reacting a compound represented by general formula (I) below (hereinafter, referred to as "compound (I)") with a base selected from the group consisting of a metal alkoxide, a metal hydride compound, an alkali metal, an organometallic compound of an alkali metal, an alkali metal amide, an alkali metal carbonate salt, an alkali earth metal carbonate salt, and a mixture of these (step 1); and
a step of obtaining an azole derivative (V) by mixing the reaction solution obtained in the step 1, a compound represented by general formula (III) below (hereinafter, referred to as "compound (III)"), and a sulfonium compound or a sulfoxonium compound in the presence of a base (step 2).

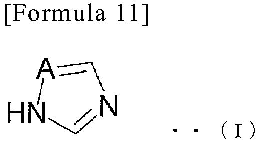

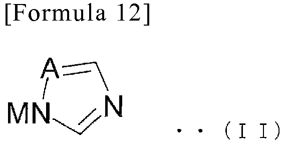

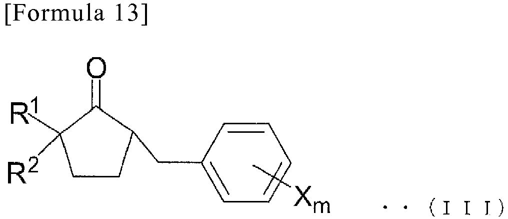

In general formula (I), A is the same as A in formula (V).

In general formula (II), M represents a metal atom contained in the base used in the step 1. Examples of M include alkali metals such as sodium, potassium, and lithium; and alkaline earth metals such as magnesium and calcium. M is preferably sodium or potassium, and more preferably sodium. A is the same as A in formula (V).

In general formula (III), R¹, R², X, and m are each the same as R¹, R², X, and m in formula (V).

A reaction scheme that schematically describes the method for producing the azole derivative (V) according to the present embodiment is illustrated in reaction scheme 1 below.

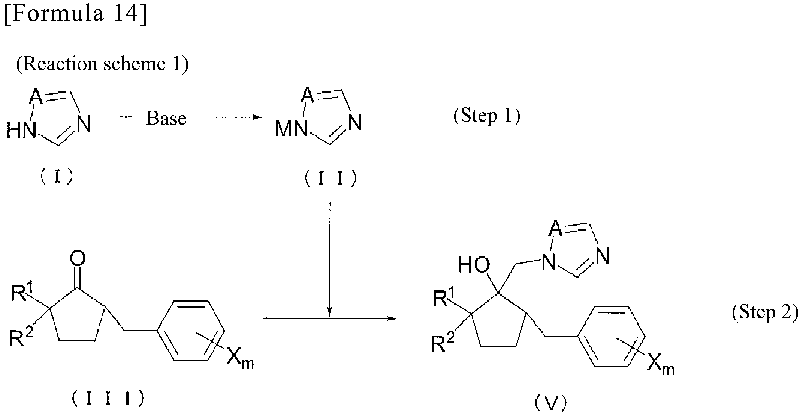

Each of the steps will be explained below.

### Step 1: Azole metal salt producing step

The step 1 is a step of obtaining a reaction solution containing a compound (II) by reacting a compound (I) with a base selected from the group consisting of a metal alkoxide, a metal hydride compound, an alkali metal, an organometallic compound of an alkali metal, an alkali metal amide, an alkali metal carbonate salt, an alkali earth metal carbonate salt, and a mixture of these (see reaction scheme 2 below).

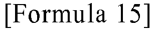

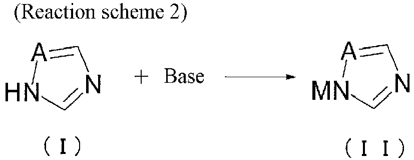

The base in the step 1 is selected from the group consisting of a metal alkoxide, a metal hydride compound, an alkali metal, an organometallic compound of an alkali metal, an alkali metal amide, an alkali metal carbonate salt, an alkali earth metal carbonate salt, and a mixture of these.

Examples of the metal alkoxide include alkali metal methoxides such as sodium methoxide and potassium methoxide; alkali metal ethoxides such as sodium ethoxide and potassium ethoxide; alkali metal butoxides such as sodium t-butoxide and potassium t-butoxide; and the like.

Examples of the metal hydride compound include sodium hydride, potassium hydride, lithium hydride, and the like.

Examples of the alkali metal include sodium, potassium, lithium, and the like.

Examples of the organometallic compound of an alkali metal include methyllithium, n-butyllithium, t-butyllithium, and the like.

Examples of the alkali metal amide include lithium diisopropylamide, sodium bis(trimethylsilyl)amide, and the like.

Examples of the alkali metal carbonate salt include sodium carbonate, sodium hydrogen carbonate, potassium carbonate, potassium hydrogen carbonate, and the like.

Examples of the alkali earth metal carbonate salt include magnesium carbonate, calcium carbonate, and the like.

Of these, the base is preferably a metal alkoxide or a metal hydride compound, more preferably a metal alkoxide, even more preferably alkali metal methoxide due to its low cost, and particularly preferably sodium methoxide. Furthermore, from the perspective of ease of handling during production, it is preferable to use the sodium methoxide in the form of a methanol solution.

The solvent in the step 1 is not particularly limited as long as the solvent does not contain water. Examples of the solvent include N,N-dimethylacetamide (DMAc), N,N-dimethylformamide (DMF), N-methylpyrrolidone (NMP), dimethyl sulfoxide (DMSO), toluene, and the like. These can be mixed and used as necessary. The solvent in the step 1 is preferably an aprotic polar solvent such as N,N-dimethylacetamide from the perspective of solubility of the base.

In the step 1, the used amount of the base is, for example, preferably from 0.2 to 5 times, and more preferably from 0.8 to 1.5 times, the molar quantity of the compound (I).

The reaction temperature and reaction time of the step 1 can be set as appropriate according to the type of solvent used, the types of compound (I) and base, and the like. The reaction temperature is, for example, preferably from 25 to 150°C, and more preferably from 60 to 90°C. The reaction time is, for example, preferably from 0.1 hours to one day, and more preferably from 0.5 hours to 10 hours.

The compound (I) may be a commercially available product or may be synthesized using a publicly known method.

### Step 2: Oxiranation / azolation step

The step 2 is a step of obtaining an azole derivative (V) by mixing the reaction solution obtained in the step 1 described above, a compound (III), and a sulfonium compound or a sulfoxonium compound in the presence of a base. The step 2 has two reactions described by reaction scheme 3 below and reaction scheme 4 below. The reaction described in reaction scheme 3 (hereinafter, referred to as "reaction A") is an oxiranation reaction that forms a compound represented by general formula (IV) (hereinafter, referred to as "compound (IV)") from the compound (III). Furthermore, the reaction described in reaction scheme 4 (hereinafter, referred to as "reaction B") is an azolation reaction that forms an azole derivative (V) from the compound (IV). In the step 2, the reaction A and the reaction B occur simultaneously and continuously in a same reaction vessel.

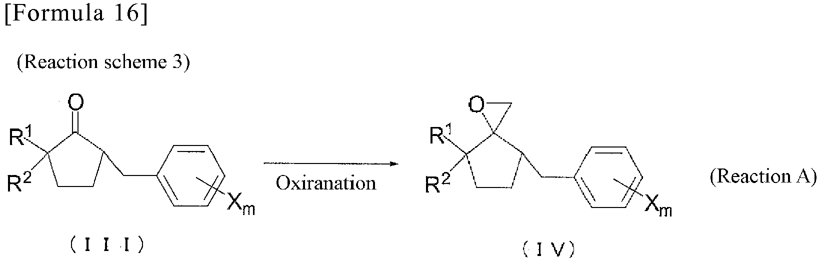

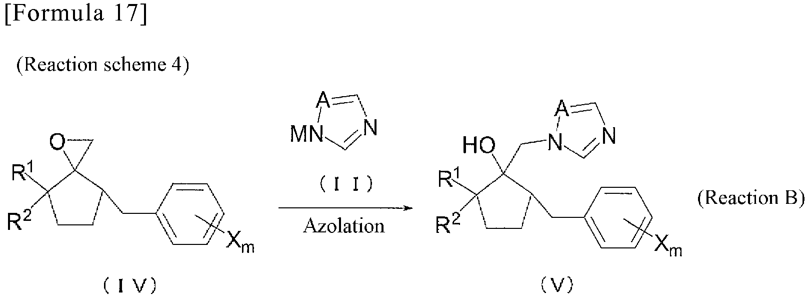

In general formula (IV), R¹, R², X, and m are each the same as R¹, R², X, and m in formula (V).

The reaction A forms the compound (IV) from the compound (III) by an action of sulfur ylide. This sulfur ylide is produced in the reaction system as a result of the base reacting with the sulfonium compound or sulfoxonium compound, and the type of sulfur ylide produced depends on the type of sulfonium compound or sulfoxonium compound used. Examples of the sulfur ylide include sulfonium methylide, such as dimethyl sulfonium methylide, and sulfoxonium methylide, such as dimethyl sulfoxonium methylide, and the like.

Examples of the sulfonium compound include trimethylsulfonium bromide, trimethylsulfonium chloride, trimethylsulfonium iodide, and the like. Examples of the sulfoxonium compound include trimethylsulfoxonium bromide (TMSOB), trimethylsulfoxonium chloride, trimethylsulfoxonium iodide, and the like. From the perspective of yield when producing the sulfur ylide, the use of a sulfoxonium compound is preferred, and of these, trimethylsulfoxonium bromide is more preferred. The sulfonium compound or sulfoxonium compound can be divided and added in separate portions.

As the base in the step 2, bases that are exemplified as the base used in the step 1 can be exemplified. In addition, as the base in the step 2, metal hydroxides, such as sodium hydroxide and potassium hydroxide, and the like can be exemplified. The base in the step 2 is preferably a metal alkoxide or a metal hydride compound, more preferably a metal alkoxide, even more preferably alkali metal methoxide due to its low cost, and particularly preferably sodium methoxide. Furthermore, from the perspective of ease of handling during production, it is preferable to use the sodium methoxide in the form of a solution. The type of the base used in the step 1 and the type of the base used in the step 2 may be the same or different. However, from the perspective of convenience, the types of the bases are preferably the same, and from the perspective of ease of handling during production, the types of the bases are more preferably a solution of sodium methoxide. Furthermore, as the base used in the step 2, the base contained in the reaction solution obtained in the step 1 may be used as is, or another base may be newly added in the step 2. Furthermore, when a base is newly added in the step 2, the base can be divided and added in separate portions.

The number of times for adding the base is not particularly limited as long as the desired objective can be achieved. The number of times is preferably from 1 to 20, and more preferably from 2 to 10. The same applies in cases where the sulfonium compound or sulfoxonium compound is divided and added in separate portions.

The compound (III) may be synthesized in accordance with the description of the reference document, WO/2012/169559, for example.

When the azole derivative (Va), which is more preferable as the azole derivative (V), is produced, a compound represented by general formula (IIIa) below (hereinafter, referred to as "compound (IIIa)") is used as the compound (III).

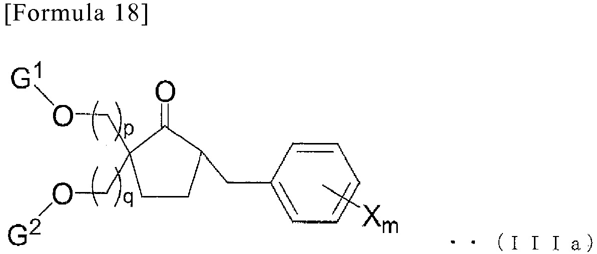

In general formula (IIIa), G¹, G², p, q, X, and m are each the same as G¹, G², p, q, X, and m in formula (Va).

When the azole derivative (Vb), which is even more preferable as the azole derivative (V), is produced, a compound represented by general formula (IIIb) below (hereinafter, referred to as "compound (IIIb)") is used as the compound (III).

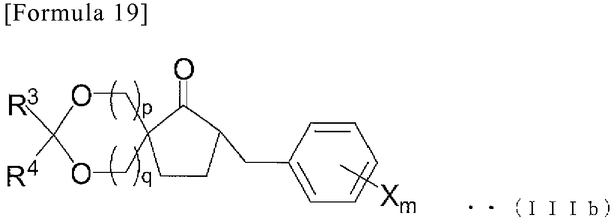

In general formula (IIIb), R³, R⁴, p, q, X, and m are each the same as R³, R⁴, p, q, X, and m in formula (Vb).

In the reaction B, the compound (IV) formed in the reaction A reacts with the compound (II), which is present in the reaction system, to form the azole derivative (V). More specifically, the azole derivative (V) is formed by a carbon-nitrogen bond being formed between a carbon atom that constitutes the oxirane ring in the compound (IV) and a nitrogen atom in the compound (II).

The reaction temperature and reaction time of the step 2 can be set as appropriate according to the types of solvent used, compound (III), sulfonium compound or sulfoxonium compound, and base, and the like. The reaction temperature is, for example, preferably from 20 to 200°C, and more preferably from 50 to 150°C. Furthermore, the reaction time is, for example, preferably from 0.1 hours to several days, and more preferably from 0.5 hours to 2 days.

The total used amount of the sulfonium compound or sulfoxonium compound is preferably from 0.5 to 5 times, and more preferably from 0.8 to 2 times, the molar quantity of the compound (III). The used amount of the compound (III) is preferably from 0.2 to 1.5 times, and more preferably from 0.5 to 1.2 times, the molar quantity of the compound (I).

In the step 2, the used amount of the base is, for example, preferably from 0.1 to 2 times, and more preferably from 0.2 to 1 time, the molar quantity of the compound (III).

As described above, the production method according to the present invention uses a base that does not require the use of water or that does not form water, as the base in the step 1. Therefore, a step of performing azeotropic dehydration is not necessary. Furthermore, the base in the step 1 can be used as the base in the step 2. Therefore, even when the reaction solution obtained in the step 1 is mixed as is with the compound (III) and the sulfonium compound or the sulfoxonium compound in the step 2, it is possible to reduce the risk of lowering the yield. In fact, as described later in Examples, the compound (V) was successfully synthesized at the same yield as that of a step 2 using a conventional method. Furthermore, since the reaction of the step 2 can proceed when the compound (III) and the sulfonium compound or the sulfoxonium compound are added to the reaction solution obtained in the step 1, the step 1 and the step 2 can be performed continuously. Therefore, the production method according to the present invention can achieve simplification of the procedure while decrease in the yield is suppressed.

### 3. Summary

As described above, the present invention is a method for producing an azole derivative represented by general formula (V) below: 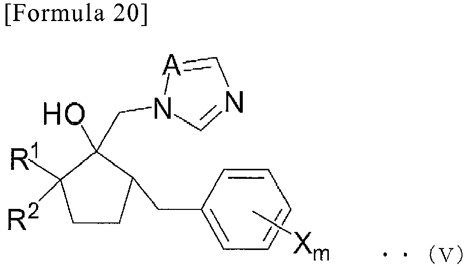 (in formula (V), R¹ and R² each independently represent a hydrogen atom, an alkyl group having from 1 to 4 carbons, or -CₙH₂ₙ-O-G; G represents a protecting group; n represents an integer of 1 to 4; R¹ and R² may together form a ring; X represents a halogen atom, an alkyl group having from 1 to 4 carbons, a haloalkyl group having from 1 to 4 carbons, an alkoxy group having from 1 to 4 carbons, a haloalkoxy group having from 1 to 4 carbons, a phenyl group, a cyano group, or a nitro group; m represents an integer of 0 to 5; when m is 2 or greater, a plurality of X may be different from each other; and A represents a nitrogen atom or a methine group); the method comprising:
a step 1 of obtaining a reaction solution containing a compound represented by general formula (II) below by reacting a compound represented by general formula (I) below with a base selected from the group consisting of a metal alkoxide, a metal hydride compound, an alkali metal, an organometallic compound of an alkali metal, an alkali metal amide, an alkali metal carbonate salt, an alkali earth metal carbonate salt, and a mixture of these 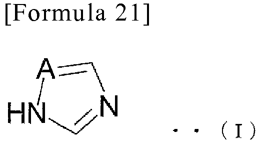 (in formula (I), A is the same as A in formula (V)) 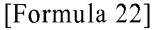 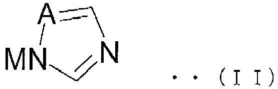 (in formula (II), M represents a metal atom contained in the base used in the step 1; and A is the same as A in formula (V)); and
a step 2 of obtaining an azole derivative represented by general formula (V) above by mixing the reaction solution, a compound represented by general formula (III) below, and a sulfonium compound or a sulfoxonium compound in the presence of a base 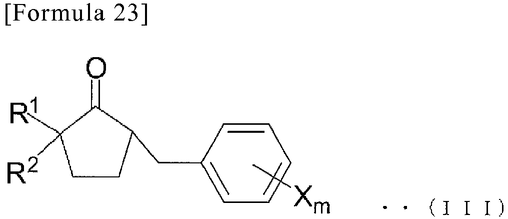 (in formula (III), R1, R2, X, and m are each the same as R1, R2, X, and m in formula (V)).

The base in the step 1 is preferably selected from the group consisting of a metal alkoxide, a metal hydride compound, and a mixture of these.

The base in the step 1 is more preferably a metal alkoxide.

The base in the step 1 is even more preferably sodium methoxide.

The base in the step 2 is preferably the same type of base as the base in the step 1.

The azole derivative represented by general formula (V) above is preferably an azole derivative represented by general formula (Va) below: 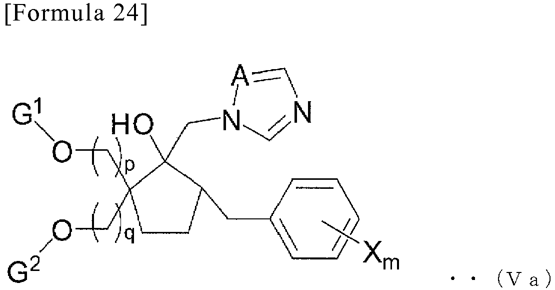 (in formula (Va), G¹ and G² each represent a protecting group, and G¹ and G² may together form a ring; p and q are each independently 1 or 2; and X, m, and A are each the same as X, m, and A in formula (V)); and
the compound represented by general formula (III) above is preferably a compound represented by general formula (IIIa) below: 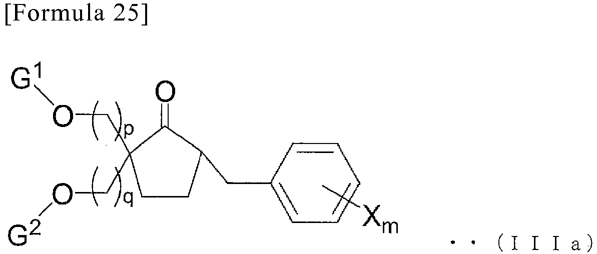 (in formula (IIIa), G1, G2, p, q, X, and m are each the same as G1, G2, p, q, X, and m in formula (Va)).

The azole derivative represented by general formula (Va) above is more preferably an azole derivative represented by general formula (Vb) below: 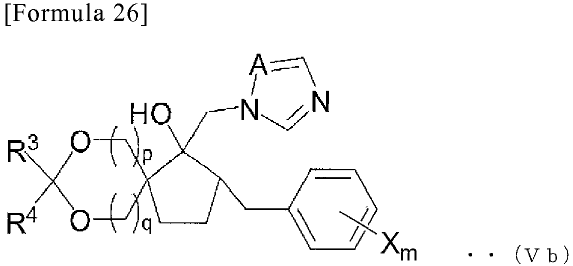 (in formula (Vb), R³ and R⁴ are each independently a hydrogen atom, an alkyl group having from 1 to 4 carbons, a phenyl group, or a benzyl group, and one or more hydrogen atoms on the phenyl group and one or more hydrogen atoms on a phenyl moiety of the benzyl group may be substituted with an alkyl group having from 1 to 4 carbons, an alkoxy group having from 1 to 4 carbons, or a halogen atom; p, q, A, X, and m are each the same as p, q, A, X, and m in formula (Va)); and
the compound represented by general formula (IIIa) above is more preferably a compound represented by general formula (IIIb) below: 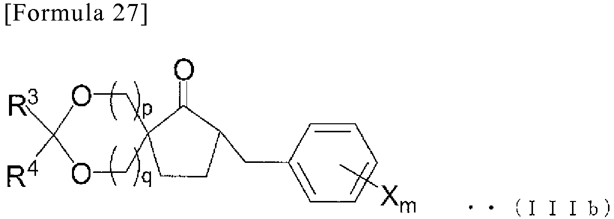 (in formula (IIIb), R3, R4, p, q, X, and m are each the same as R3, R4, p, q, X, and m in formula (Vb)).

R³ and R⁴ are even more preferably each independently a hydrogen atom or an alkyl group having from 1 to 4 carbons.

X is preferably a fluorine atom or a chlorine atom, and m is preferably 0 or 1.

Embodiments of the present invention will be described in further detail hereinafter using examples. Of course, the present invention is not limited to the examples below, and it goes without saying that various modes are possible with regard to the details thereof. Furthermore, the present invention is not limited to the embodiments described above, and various modifications are possible within the scope indicated in the claims. Embodiments obtained by appropriately combining the technical means disclosed by the embodiments are also included in the technical scope of the present invention. In addition, all of the documents disclosed in the present specification are hereby incorporated by reference.

### EXAMPLES

Production Example 1: Synthesis of 2-(4-chlorobenzyl)-8,8-dimethyl-1-(1H-1,2,4-triazol-1-ylmethyl)-7,9-dioxaspiro[4,5]decan-1-ol (compound V-1; azole derivative (Vb) in which R³ = CH₃, R⁴ = CH₃, p = 1, q = 1, Xₘ = 4-Cl, A = N)

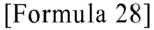

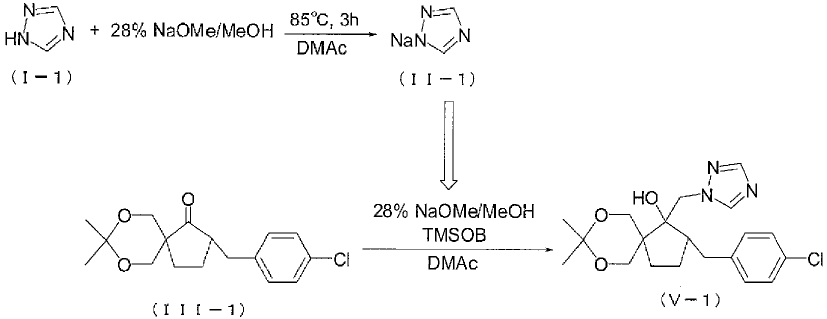

In dehydrated N,N-dimethylacetamide (6.18 mL), 1,2,4-triazole (compound I-1; compound (I) in which A = N) (15.0 mmol) was dissolved and the mixture was heated to 85°C. Thereafter, a 28% sodium methoxide methanol solution (15.0 mmol) was added thereto, and the mixture was heated and stirred at 85°C for 3 hours. To the obtained reaction solution, 2-(4-chlorobenzyl)-8,8-dimethyl-7,9-dioxaspiro[4,5]decan-1-one (compound III-1; compound (IIIb) in which R³ = CH₃, R⁴ = CH₃, p = 1, q = 1, Xₘ = 4-Cl) (10.0 mmol) and TMSOB (14.0 mmol) were then added, and a 28% sodium methoxide methanol solution (5.0 mmol) was divided and added in separate portions. After the mixture was heated and stirred at 85°C for 100 minutes, the mixture was cooled to the room temperature, water was added, and extraction was performed using ethyl acetate. The organic layer was washed with water and a saturated salt solution, and dried with anhydrous sodium sulfate. The crude product was purified by means of silica gel chromatography, and the target compound V-1 was obtained.
Product: 3.14 g
Purity: 97.7%
Yield: 78.1% (yield of the compound V-1 relative to the amount of the compound III-1) Properties: white solid

### Reference Example 1: Synthesis of compound V-1 using sodium hydroxide

In N,N-dimethylacetamide (120 mL), 1,2,4-triazole (compound I-1) (0.390 mol) was dissolved. Thereafter, water (7 mL) and sodium hydroxide (0.390 mol) was added thereto, and the mixture was heated to 80°C and dissolved. Toluene (144 mL) was added, and water was removed by means of azeotropic dehydration by further increasing the temperature. The obtained reaction solution was cooled to 65°C, N,N-dimethylacetamide (75 mL) and a crude material of the compound III-1 (in terms of pure content, 0.300 mol) were added, and then TMSOB (0.360 mol) and a 28% sodium methoxide methanol solution (0.18 mol) were divided and added in separate portions. After the mixture was heated and stirred at 65°C for 11 hours, a separately prepared triazole sodium salt (0.060 mol) was added to react the unreacted compound III-1. After 4 hours, the mixture was cooled to the room temperature, water was added, and extraction was performed using toluene. The organic layer was washed with water to obtain a crude solution of the target compound V-1. Yield in terms of pure content was determined by quantitative analysis.
Product: 398.85 g
Purity: 21.2%
Yield in terms of pure content: 71.9% (yield of the compound V-1 relative to the amount of the compound III-1)

### Industrial Applicability

The present invention can be suitably used for the production of a compound that can be used as an active ingredient of agricultural and horticultural fungicides.

## Claims

1. A method for producing an azole derivative represented by general formula (V) below: 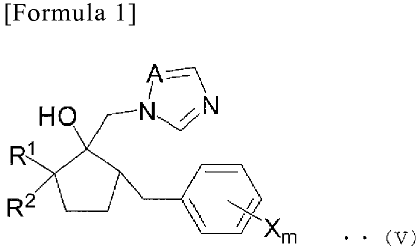 wherein, R1 and R2 each independently represent a hydrogen atom, an alkyl group having from 1 to 4 carbons, or -CnH2n-O-G; G represents a protecting group; n represents an integer of 1 to 4; R1 and R2 may together form a ring; X represents a halogen atom, an alkyl group having from 1 to 4 carbons, a haloalkyl group having from 1 to 4 carbons, an alkoxy group having from 1 to 4 carbons, a haloalkoxy group having from 1 to 4 carbons, a phenyl group, a cyano group, or a nitro group; m represents an integer of 0 to 5; when m is 2 or greater, a plurality of X may be different from each other; and A represents a nitrogen atom or a methine group;
the method comprising:
a step 1 of obtaining a reaction solution containing a compound represented by general formula (II) below by reacting a compound represented by general formula (I) below with a base selected from the group consisting of a metal alkoxide, a metal hydride compound, an alkali metal, an organometallic compound of an alkali metal, an alkali metal amide, an alkali metal carbonate salt, an alkali earth metal carbonate salt, and a mixture of these; 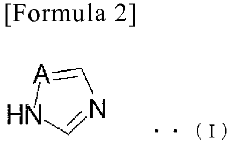 wherein, A is the same as A in formula (V); 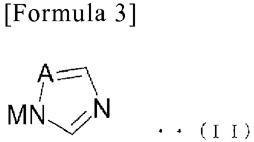 wherein, M represents a metal atom contained in the base used in the step 1; and A is the same as A in formula (V); and
a step 2 of obtaining an azole derivative represented by general formula (V) above by mixing the reaction solution, a compound represented by general formula (III) below, and a sulfonium compound or a sulfoxonium compound in the presence of a base; 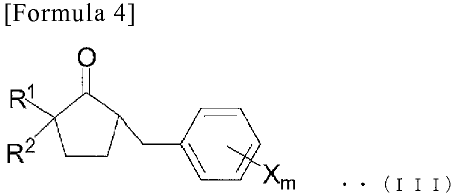 wherein, R¹, R², X, and m are each the same as R¹, R², X, and m in formula (V).

2. The method for producing an azole derivative according to claim 1, wherein the base in the step 1 is selected from the group consisting of a metal alkoxide, a metal hydride compound, and a mixture of these.

3. The method for producing an azole derivative according to claim 2, wherein the base in the step 1 is a metal alkoxide.

4. The method for producing an azole derivative according to claim 3, wherein the base in the step 1 is sodium methoxide.

5. The method for producing an azole derivative according to any one of claims 1 to 4, wherein the base in the step 2 is the same type of base as the base in the step 1.

6. The method for producing an azole derivative according to any one of claims 1 to 5, wherein the azole derivative represented by general formula (V) above is an azole derivative represented by general formula (Va) below: 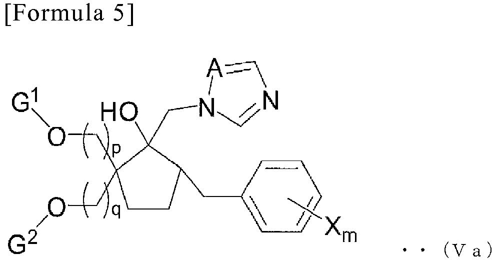 wherein, G¹ and G² each represent a protecting group, and G¹ and G² may together form a ring; p and q are each independently 1 or 2; and X, m, and A are each the same as X, m, and A in formula (V); and
the compound represented by general formula (III) above is a compound represented by general formula (IIIa) below: 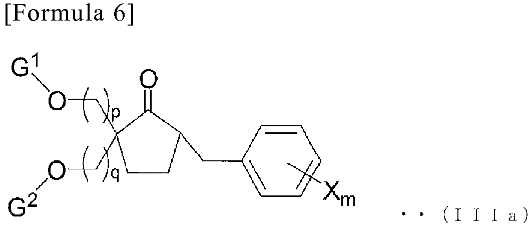 wherein, G¹, G², p, q, X, and m are each the same as G¹, G², p, q, X, and m in formula (Va).

7. The method for producing an azole derivative according claim 6, wherein the azole derivative represented by general formula (Va) above is an azole derivative represented by general formula (Vb) below: 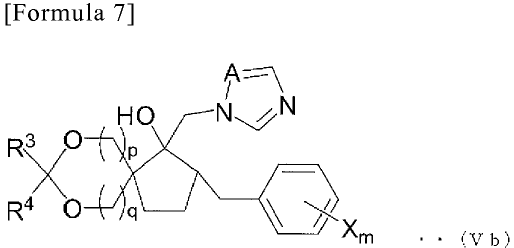 wherein, R³ and R⁴ are each independently a hydrogen atom, an alkyl group having from 1 to 4 carbons, a phenyl group, or a benzyl group, and one or more hydrogen atoms on the phenyl group and one or more hydrogen atoms on a phenyl part of the benzyl group may be substituted with an alkyl group having from 1 to 4 carbons, an alkoxy group having from 1 to 4 carbons, or a halogen atom; p, q, A, X, and m are each the same as p, q, A, X, and m in formula (Va); and
the compound represented by general formula (IIIa) above is a compound represented by general formula (IIIb) below: 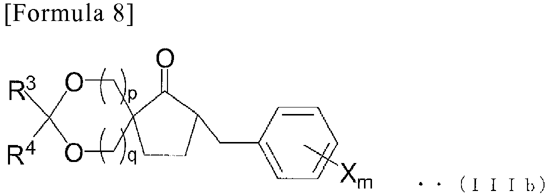 wherein, R³, R⁴, p, q, X, and m are each the same as R³, R⁴, p, q, X, and m in formula (Vb).

8. The method for producing an azole derivative according to claim 7, wherein R³ and R⁴ are each independently a hydrogen atom or an alkyl group having from 1 to 4 carbons.

9. The method for producing an azole derivative according to any one of claims 1 to 8, wherein X is a fluorine atom or a chlorine atom, and m is 0 or 1.
